# DEMANDE DE BREVET EUROPEEN

(11) **EP 1 211 303 A1**
(43) Date de publication de la demande: **05.06.2002**
(21) Numéro de dépôt: 01403020.9
(22) Date de dépôt: 26.11.2001
(51) Int. Cl.: C11B 1/04, C11B 1/06, A23N 1/02, A23D 9/00, A23L 1/30, A61K 31/23, A61K 7/00, A23K 1/14

(54) **Procédé et appareil de valorisation intégrale des drupes oléifères, en particulier des olives et les produits spécifiques obtenus**

(30) Priorité: 01.12.2000 FR 0015558
(71) Demandeur: Martel, Jean-Pierre, F-06250 Mougins (FR); Farcot, Olivier, 04110 Reillamme (FR)
(72) Inventeur: Martel, Jean-Pierre, F-06250 Mougins (FR); Farcot, Olivier, 04110 Reillamme (FR)
(74) Mandataire: Portal, Gérard

(57) **Abrégé**

L'invention concerne un procédé et un appareil de traitement de drupes oléifères.

Cet appareil de traitement de drupes oléifères est caractérisé en ce qu'il comprend :
a) au moins un dispositif de traitement (20, 24) des drupes entières qui prépare une déstructuration physique des drupes entières avec amorce de décollage de la pulpe des drupes relativement aux peaux et des noyaux, dans des conditions évitant sensiblement l'oxydation, notamment principalement des anti-oxydants naturels de la pulpe ;
b) au moins un dispositif (42, 52 ; 110 ; 120, 124, 130, 134, 160, 170) de séparation physique et de récupération d'une part (en 100) des pulpes essentiellement exemptes des noyaux et avantageusement des peaux ; d'autre part (en 92, 96, 110) des noyaux essentiellement exempts de pulpe, avantageusement avec les peaux, de préférence dans des conditions évitant sensiblement l'oxydation ;
c) au moins un dispositif (160, 170, 172, 174) de séparation et de récupération de l'huile de la pulpe, essentiellement exempte de l'huile du noyau et contenant les anti-oxydants naturels de la pulpe, en améliorant la résistance à l'oxydation de l'huile de la pulpe.

L'invention permet d'obtenir une huile de la pulpe essentiellement pure, ainsi que de récupérer les autres constituants des drupes entières. L'invention est de préférence appliquée au traitement des olives.

## Description

L'invention concerne essentiellement un procédé et un appareil de traitement des fruits entiers de types drupes oléifères, ainsi que les produits qui en sont issus, et notamment une pure huile de pulpe, une pulpe entière ou partiellement déshuilée et des arômes volatils spécifiques du fruit ainsi qu'une valorisation des co-produits (bois, peaux et amande) rendue possible du fait de l'invention.

Les drupes sont entendues selon l'invention d'un point de vue botanique comme étant des fruits à noyau, c'est à dire des fruits comprenant de l'extérieur vers l'intérieur, un épicarpe vulgairement dénommé peau, un mésocarpe charnu souvent vulgairement dénommé pulpe ou chair, et l'endocarpe vulgairement dénommé noyau qui est formé d'une coque ligneuse ou bois, et protège la graine habituellement dénommée amande contenue à l'intérieur de la coque.

L'invention s'applique notamment aux drupes oléifères (contenant de l'huile à la fois dans le mésocarpe et dans l'amande de l'endocarpe) telles que l'olivier (Oléa Europa), le palmier à huile (Elacis Guinéesensis) et l'avocat (Perséa Américana) sans exclusion des autres drupes oléifères.

De manière générale l'invention concerne tous les fruits de types drupes oléifères pour lesquels la séparation et la préparation du mésocarpe en dehors des autres constituants du fruit (épicarpe et endocarpe) constituent une amélioration soit :
- 1) en vue de l'extraction de produits spécifiques contenus dans le mésocarpe tel que les produits huileux et d'une valorisation des deux produits ainsi obtenus (huile et pulpe partiellement déshuilée) après séparation.
- 2) soit pour une utilisation du mésocarpe ou pulpe entière.
- 3) soit de préférence d'une valorisation conséquente de tous les produits issus du fruit entier de l'épicarpe à l'endocarpe et incluant les bois et amandes.

Dans le cadre de l'invention, un autre but est de réaliser la séparation et la différenciation des différentes parties du fruit entier tout en respectant leur intégrité dans le but de valoriser leurs constituants et de mettre à profit leurs propriétés propres dans des utilisations de type énergétique, industrielle, alimentaire, cosmétique ou pharmaceutique. L'invention permet d'éviter que le mélange de la technique antérieure préalable à l'extraction d'huile et l'indifférenciation des différentes parties du fruit en fassent des effluents polluants devant faire l'objet de traitement spécifique constituant une déséconomie pour l'entreprise.

### Description de l'art antérieur

Depuis l'antiquité, les olives sont broyées entières et longuement malaxées à l'air libre avant d'être soumises à la pression pour l'extraction de leur huile.

L'emploi de séparateurs centrifuges est actuellement connu pour séparer les phases solides, liquides et aqueuses. Les derniers développement comprennent en amont des broyeurs métalliques complétés par des dilacérateurs et des malaxeurs fonctionnant à l'air libre pendant des durées de l'ordre de 30 à 40 minutes et dans des bacs semi-cylindriques munis d'une double paroi avec un dispositifs de réchauffage amenant la pâte d'olive entière broyée à des températures comprises entre 25 et 30°C.

Les inconvénients présentés par les modes de fabrication classiques sont énumérées ci-dessous :
Lors du broyage, que ce soit dans les meules traditionnelles ou des broyeurs rotatifs modernes, la présence de l'air entraîne une oxydation de l'huile libérée des cellules, aggravée par la durée de l'opération dans le premier cas, ou par le courant d'air induit par une rotation rapide dans le second. L'oxydation est responsable de la dégradation de la qualité de l'huile au fur et à mesure de sa durée de conservation.

Quant aux malaxeurs et triturateurs, leur action prolongée produit des phénomènes d'oxydation à l'intérieur même de la pâte.

Le broyage et le malaxage du fruit entier sur un temps long, en présence d'air et à température comprise entre 28 et 30° entraîne une hydrolyse due à la forte teneur en eau du mésocarpe, une lipolyse enzymatique ainsi qu'une lipolyse microbienne liée à la présence d'une microflore sur l'épicarpe. En effet le temps qui s'écoule entre le broyage des olives et celui de la séparation de l'huile avec l'eau n'est pas assez bref pour éviter ces phénomènes qui entraînent une dégradation de l'huile.

Plus encore ce temps long (30 à 40 minutes) à des températures relativement élevées entraîne la perte des arômes les plus volatils du fruit et qui font partis de l'architecture organoleptique propre à chaque variété d'olive.

De plus le mélange préalable de tous les composants du fruit par broyage entraîne plusieurs inconvénients :
L'huile de pulpe se trouve en contact ou mélangée avec les graisses contenues dans l'épicarpe, l'huile du bois, et l'huile de l'amande, toutes plus acides et plus sujettes au rancissement. Si les deux premières peuvent contribuer à une détérioration des saveurs et des goûts propre à l'huile de pulpe, l'huile contenue dans l'amande trouve des applications en pharmacie et en cosmétique pour des valeurs plus grandes que l'huile de pulpe.

Outre la perte de valorisation dans chacun de leur domaine des composants de l'épicarpe et de l'endocarpe le mélange appelé grignons devient un effluent solide qui même après une éventuelle extraction par solvant de l'huile résiduelle constitue à l'heure actuel un problème quand à son recyclage propre. De même les lavages à l'eau sont responsables non seulement de la perte de principe hydrophiles nécessaires à la bonne conservation de l'huile tel que certains polyphénols, mais génèrent des effluents dénommés margines aggravant le problème de pollution déjà posé par l'eau de végétation.

Différents procédés ont été proposés en vue de la séparation mécanique et hydraulique préalable des noyaux mais ils sont très longs et favorisent l'oxydation. En outre, ils présentent les inconvénients du triturage et du malaxage de la pâte qui s'en suit, même si ils ne s'appliquent qu'à la pulpe, et de l'augmentation des rejets d'effluents liquides.

Enfin il faut remarquer que le caractère saisonnier de l'industrie oléicole, la grande variabilité de la production ainsi que la limite de capacité horaire des équipements actuels classiques ne permettent pas l'installation de moulins capables d'absorber toutes les livraisons journalières. Il est donc obligatoire d'assurer le stockage des olives fraîches et entières sans que dans le fruit lui même se produisent des hydrolyses spontanées et des mécanismes de lipolyse enzymatique tels que décrits plus haut au cours du traitement. Or, dans la pratique et au plus haut de la récolte, les olives ne sont traitées qu'après plusieurs jours après leur récolte et stockées dans des conditions qui mettent en oeuvre les mécanismes de dégradation décrit plus haut et dont il est admis qu'ils sont en grande partie responsables de l'altération (acidification et rancissement) et de la baisse des rendements en huile.

### BUTS DE L'INVENTION DANS SON APPLICATION GENERALE AUX DRUPES OLEIFERES ET EN PARTICULIER AUX OLIVES :

L'invention a pour but principal de répondre à l'ensemble des problèmes posés par l'art antérieur tels que décrits plus haut.

L'invention a encore pour but principal de proposer un nouveau concept industriel de traitement des drupes oléifères entières, en particulier de l'olive, qui consiste à passer d'une industrie centrée sur l'extraction unique de l'huile du fruit à une industrie de traitement global du fruit entier en vue d'une valorisation intégrale des composants du fruit sans être entièrement dépendant du caractère saisonnier du fruit entier et de son traitement en frais au plus proche de sa récolte.

L'invention a encore pour but principal de résoudre le nouveau problème technique consistant en la fourniture d'une solution permettant d'améliorer les qualités organoleptiques des huiles de drupes oléifères, en particulier d'olives, leur durée de conservation, leur polyvalence de traitement et de façon générale la valorisation des différentes parties des drupes oléifères, en particulier des olives, des drupes de palmiers à huile et des drupes d'avocats, naturellement sans exclusion des autres drupes oléifères.

La présente invention a encore pour but principal de résoudre le nouveau problème technique consistant en la fourniture d'une solution qui permette de traiter les drupes oléifères entières, en particulier des olives, pour en extraire notamment leurs huiles, selon un mode de fabrication qui résulte en la réduction ou la suppression des effluents, la valorisation des sous produits, ainsi que de préférence favorisant l'autosuffisance en besoin calorifique de l'unité de production.

L'ensemble de ces buts est résolu pour la première fois dans le cadre de la présente invention, d'une manière simple, sûre et fiable, utilisable à l'échelle industrielle.

L'invention permet en outre d'obtenir des sous-produits qui peuvent être utilisés soit comme ingrédients pour produits alimentaires, pour l'alimentation humaine ou animale, en particulier pour le bétail, soit pour la fabrication de produits cosmétiques ou pharmaceutiques, soit encore comme matériaux de combustion à partir des fibres ou bois des noyaux.

Ainsi, selon un premier aspect, la présente invention fournit un procédé de traitement de drupes oléifères tel que défini à la revendication 1.

Des caractéristiques avantageuses de ce procédé de traitement sont également définies dans les sous-revendications de procédé, l'ensemble de ces revendications étant incorporé dans leur intégralité dans la description par référence.

De manière générale l'invention concerne un procédé d'obtention de tels produits consistant après lavage des drupes oléifères entières en un pré-chauffage avantageusement par micro-ondes sans exclusion des autres modes de chauffage (conduction par fluide thermique ou par résistances électriques ou par vapeur directe basse pression) afin d'atteindre un échauffement limité à la pulpe (mésocarpe), de manière rapide évitant tous phénomènes de cuisson (couple temps/température), permettant à la fois une destruction sensiblement complète de la flore microbienne contenue sur l'épicarpe ou peau du fruit ainsi que la désactivation des phénomènes enzymatiques (hydrolyse et lipolyse). Pour les micro-ondes, qui n'interviennent que sur l'eau, l'action sur la flore périphérique est favorisée par la pellicule résiduelle d'eau de lavage.

Le préchauffage des fruits est préférentiellement effectué dans une enceinte de préchauffage comprenant un tunnel à micro-onde par rapport aux autres modes de chauffage : En effet sur un fruit frais et dont l'humidité est égale ou supérieur à 50% les micro ondes sont beaucoup plus rapides pour provoquer l'échauffement voulu. Le contrôle du front de chaleur dans le mésocarpe ou pulpe et la maîtrise des phénomènes de cuisson, sont plus précis, et de plus et d'importance pour ce type de produit, le chauffage s'applique préférentiellement au molécules d'eau préservant ainsi les autres constituants de la pulpe et notamment l'huile. Cependant pour des raisons d'économie et de puissance électrique installée il est possible que le chauffage direct par vapeur détendue ou indirect par fluide thermique ou résistance électrique soient préférables pour des quantités horaires élevées (au dessus de 1 T/h). De plus la récupération des bois des noyaux autorise, dans ce cas, une autonomie sur le site de fabrication de chaleur. Cependant ils peuvent tout aussi bien servir comme combustible pour entraîner un générateur électrique.

Au sortir du tunnel continu de préchauffage les drupes sont introduites dans une enceinte sous pression réduite dans le but de produire l'éclatement des tissus cellulaires de la pulpe des drupes. Dans cette enceinte sous pression réduite, on réalise de préférence selon l'invention et avantageusement dans sa partie inférieure, une séparation mécanique par un dispositif mécanique de séparation qui complète la destructuration des drupes en complétant le décollement des différents composés du fruit, peau, pulpe et noyau et une séparation fine de ceux-ci, peau et noyau restant entiers, par effet mécanique et on réalise la séparation mécanique sous la même pression réduite, donc à l'abri de l'oxygène.

L'enceinte est maintenue à une pression inférieure à la pression atmosphérique, de préférence inférieure à 100 hectopascals encore mieux comprise entre 50 et 100 hectopascals.

Ainsi, le préchauffage précité permet d'établir un différentiel de pression et de température dans l'enceinte sous pression réduite, formant de préférence aussi une raffineuse, favorable à la destruction des tissus cellulaires de la pulpe, au décollement des différents composés du fruit (peau, pulpe et noyau) et une séparation fine de ceux-ci. (peau et noyau restant entiers).

Le vide est assuré notamment par un groupe générateur de vide de préférence situé en aval du système de récupération et de concentration des eaux aromatiques issues de la vaporisation d'une partie des eaux de végétation principalement des pulpe des drupes.

L'enceinte comprend avantageusement en outre un dispositif mécanique de séparation des noyaux et peaux, relativement à la pulpe, comprenant, par exemple, un tambour rotatif filtrant à fentes ou à trous par exemple de largeur comprise entre 1 à 2 mm et de diamètre par exemple de 2 à 3 mm.

Autrement dit, le mode de réalisation préféré de l'invention est que le dispositif mécanique de séparation soit disposé à l'intérieur de l'enceinte sous pression réduite.

De manière moins avantageuse, il est possible en variante d'intercaler une enceinte sous pression réduite entre l'appareil de pré-chauffage et le dispositif de séparation ou raffineuse. Mais on perd dans ce cas l'effet instantané et simultané, on augmente l'encombrement et les coûts d'équipement, et on s'oblige à remédier à l'effet d'entraînement des particules de pulpe dans le circuit de condensation/concentration. Ainsi, selon l'invention, de préférence l'éclatement des fruits et la séparation physique des noyaux et peaux est réalisé dans une seule enceinte sous pression réduite, à l'abri de l'oxydation de l'air.

De même la raffineuse peut être constituée de tout autre mode physique de séparation. Mais là aussi on se prive d'un effet essentiel lié au tambour rotatif dont les racleurs et les pores permettent et de manière simultanée d'effectuer une séparation de la pulpe et un affinage de celle-ci afin d'obtenir une "purée" apte à une extraction directe de l'huile contenue dans la pulpe.

Dans une exécution différente les racleurs tournent à l'intérieur d'un tambour fixe. On emploi le terme de raffineuse pour désigner cet appareil propre à l'invention mais celui-ci va bien au delà d'une raffineuse classique. Il s'agit là d'une raffineuse à double effet : un effet en deux étapes quasi instantanées de préparation de la pulpe par éclatement cellulaire suivie d'un affinage par la combinaison du tambour alvéolé et des racleurs et d'un effet de séparation des constituants du fruit (peau, noyau et pulpe).
A cette étape un certain nombre de phénomènes essentiels se produisent de manière instantanée et simultanée et qui sont liés à l'invention.
1) Sous l'effet du différentiel de pression, l'eau du mésocarpe se vaporise provoquant l'éclatement et la destruction des membranes des tissus cellulaires de la pulpe des drupes.
2) La rupture des liaisons entre la peau, la pulpe et le noyau des drupes.
3) La séparation de ces trois composants des drupes et dans le respect de l'intégrité des peaux et de la pulpe (pulpe d'un coté et peau/noyau de l'autre).
4) Le refroidissement instantané du végétal.
5) L'évaporation des fractions les plus volatiles du fruit par entraînement lié à la vaporisation de l'eau. Il est à noter que l'évaporation de l'eau conduit à une perte de l'ordre de 8 à 10 % en poids de l'ensemble pulpe + peau. En outre, cette évaporation conduit à un refroidissement quasi-instantané de la pulpe et de la peau préalablement préchauffées.

Le procédé est avantageusement effectué en continu jusqu'à l'obtention des différents produits, il peut tout aussi bien être partiellement en discontinu, notamment pour ce qui concerne le traitement de la peau et du noyau, ou de la pulpe (leur déshydratation plus ou moins partielle permettant un stockage d'attente sans risque de dégradation) et moins avantageusement si le discontinu concerne l'ensemble des opérations.

La peau et le noyau du fruit sont récupérés au sortir de la raffineuse et amenés dans un bac de réception par tout moyen connu, de préférence à vis excentrée, assurant étanchéité de manière à ne pas couper la pression réduite présente dans l'enceinte sous pression réduite contenant le dispositif de séparation ou raffineuse.

De même, la pulpe sous forme de "purée" est récupérée en bas du cône de la raffineuse et transférée à l'extérieur de la raffineuse à l'aide d'une pompe pour alimenter les équipement d'aval soit en vue d'une séparation des phases huileuse, aqueuses et solides, soit en vue d'une utilisation entière. La pompe peut être de type classique si les équipement d'aval sont maintenus dans la même pression, dans le cas contraire on utilise une pompe qui permet de préserver la même pression continue dans le séparateur de phases sachant que le produit sous forme de pâte permet un effet de "bouchon" entre les deux milieux. Entre sa sortie de la raffineuse et sa reprise par les équipements d'aval on peut préférentiellement introduire un échangeur de façon à régler la température de la pâte du mésocarpe ainsi préparée à la température désirée pour toutes opérations futures sur cette dernière. De même au sortir de la raffineuse on prévoit pour la pulpe entière un sécheur, notamment de type tambour rotatif à racloir afin de déshydrater la pulpe soit, pour une extraction de l'huile par pression (on a ainsi par évaporation éliminé la phase aqueuse libre), soit pour sortir une pulpe entière et stabilisée.

Au sortir de cette étape on obtient un ensemble de produits :
a) Une pulpe sous forme de purée exempte de peaux et de noyaux. Celle-ci étant utilisable telle quelle ou déshydratée et pouvant être passée par tous systèmes de décantation ou d'extraction connu afin de séparer les phases huileuses relativement aux phases aqueuses et aux phases solides. Ceux-ci sont avantageusement tenus dans la même chaîne de pression sous atmosphère contrôlée afin d'éviter tous phénomènes d'oxydation. Il peut, mais de manière moins avantageuse être en dehors du circuit d'atmosphère contrôlée tout en étant maintenu sous atmosphère inerte. Ils peuvent aussi être comme dans l'industrie actuelle sans contrôle d'atmosphère dans la mesure où la séparation rapide (de type presse ou décanteur/centrifugeuse) n'altère pas la qualité des produits.
   En cas de séparation, ce qui est la voie classique on obtient une pure huile de pulpe au plus proche de sa naturalité dans le fruit (conservation des caractéristiques organoleptiques, des antioxydants naturels et des vitamines) ainsi qu'une purée partiellement déshuilée, contenant 3 à 6 % d'huile, exempte de peaux, de noyaux ou d'amande, valorisable en alimentation humaine et à moindre degré de valeur en alimentation animale.
   Il faut noter que la valorisation nouvelle de cette purée permet de piloter la quantité d'huile extraite à son rendement optimal eu égard à la qualité de l'huile. En effet on sait que pour sortir les derniers pourcentage d'huile on est obligé de mettre en oeuvre des techniques plus lourdes (pression, ajout d'eau chaude, vitesse de rotation) qui ont pour effet de dégrader ces derniers pourcentages d'huiles et qui sont mélangés, de fait, à l'ensemble de l'huile extraite. Hors et compte tenu de la valeur de cette pulpe partiellement déshuilée il est possible de conserver un taux supérieur (de l'ordre de 10 à 15 %) à l'intérieur de celle-ci en fonction de la demande du marché et dans laquelle l'huile gardera toute ses qualités d'origine naturelle.
   En cas de non séparation on obtient une purée entière contenant l'ensemble des composants de la pulpe et utilisable comme base alimentaire humaine. C'est ainsi le cas pour une purée d'olive ou d'avocat mais qui, à l'heure actuelle, sont obtenues par des voies techniques beaucoup plus lourdes, dans des industries différentes et toujours selon des procédés mécaniques qui induisent un effet d'oxydation néfaste à la qualité et à la conservation du produit final.
b) Un mélange de la peau et du noyau extrait de la raffineuse par tout système permettant de maintenir une basse pression dans la raffineuse et avantageusement un système par sas multiple continu.
   Après un séchage rapide sur tambour, ou tout autre moyen de séchage, les peaux entières sont séparées des noyaux entiers sur un séparateur ventilateur cyclonique ou tout autre système connu. En fonction du type de drupe les peaux peuvent faire l'objet de tous types de valorisation en l'état ou par extraction de composant spécifique et notamment les cires.
   Les noyaux sont cassés de façon à séparer les bois de l'amande qui doit rester entière de façon à préserver son intégrité assurée par son épiderme. Les bois pouvant faire l'objet de tous types de valorisation (énergétique, charges alimentaires, industrielles etc.). Les amandes peuvent être avantageusement pressées pour en extraire une huile (souvent en proportion importante de l'ordre de 40%) de haute valeur alimentaire cosmétique ou pharmaceutique telle que l'huile d'amandon d'olive ou l'huile de palmiste. Les tourteaux issus du pressage qui représentent un produit à haute valeur alimentaire et qui comme dans le cas du tourteau d'olive peuvent contenir des principes actifs utilisables en phytothérapie présent (oleuropéine contenue dans les feuilles et que l'on peut plus facilement concentrer).
c) Les arômes les plus volatils de la drupe, entraînés par la vaporisation d'une partie de l'eau de la pulpe à l'intérieur de la raffineuse sous vide partiel, sont récupérés dans un condenseur à contre courant et concentré au niveau du "groupe générateur de vide". Ces arômes, les plus fins et les plus volatils du fruit sont généralement perdus dans les traitements classiques au cours du malaxage long et dans une température comprise entre 28 et 30°. Récupérés et concentrés ils peuvent être utilisés tels quels ou réintroduits dans l'huile ou dans la pulpe entière ou partiellement déshuilée, selon le goût des consommateurs ou l'exigence du marché.

Selon un deuxième aspect, la présente invention fournit encore un appareil de traitement de drupes oléifères entières, en particulier d'olives, de drupes de palmiers à huile, de drupes d'avocats, tel que défini dans la revendication indépendante d'appareil ainsi que, selon des modes de réalisation avantageux, dans les sous-revendications d'appareil.

D'autre part, toutes les variantes de réalisation du procédé qui impliquent des caractéristiques d'appareillage, sont aussi couvertes dans le cadre de l'appareil selon l'invention.

L'invention couvre aussi tous les produits obtenus par le procédé ou l'appareil selon l'invention.

L'invention couvre encore une huile de pulpe de drupes oléifères, en particulier d'huile de pulpe d'olive, une huile de drupes de palmiers à huile, une huile de drupes d'avocats, de pureté améliorée, caractérisée en ce qu'elle est essentiellement exempte d'huile d'amande contenue dans le noyau, en ce qu'elle contient des antioxydants naturels présents dans l'huile à l'état natif, notamment en étant préparée à l'abri de l'oxydation, en particulier à l'abri de l'oxygène de l'air, cette huile de mésocarpe ou pulpe étant susceptible d'être obtenue par le procédé tel que précédemment défini ou par l'appareil tel que précédemment défini.

Selon un mode de réalisation avantageux, l'huile de pulpe de drupes est encore caractérisée en ce qu'elle contient aussi au moins en partie les composés les plus volatils de l'odeur naturelle du fruit, notamment en ayant été réintroduits dans l'huile finale, cette huile contenant de préférence la majorité des polyphénols naturels, et du tocophérol présent dans le fruit frais entier ; avantageusement la pure huile présentant une acidité exprimée en acide gras, de préférence en acide oléique, inférieure à 1 % après deux ans de stockage à température ambiante, de préférence une acidité exprimée en acide gras, de préférence oléique, inférieure à 0,7 g/100 g d'huile, après deux ans de stockage à température ambiante.

L'invention couvre encore comme produit l'huile d'amande contenue dans le noyau de drupes, et en particulier l'huile d'amande des olives ou d'autres drupes telles que drupes de palmiers à l'huile et drupes d'avocats, telles que susceptibles d'être obtenues par la mise en oeuvre du procédé précédemment décrit ou de l'appareil précédemment décrit, ainsi que du mode de réalisation préféré donné à titre d'exemple.

L'invention couvre encore une pâte de pulpe de drupes telle que susceptible d'être obtenue par le procédé ou par l'appareil tel que décrit dans la présente description prise en son ensemble, incluant l'exemple.

L'invention couvre encore les composés aromatiques volatils récupérés des drupes et en particulier des olives, des drupes du palmier à huile ou des drupes d'avocat, tels que susceptibles d'être obtenus par le procédé ou l'appareil selon la présente invention tel que décrit dans la description prise dans son ensemble ; ainsi que leur utilisation pour aromatiser les huiles précitées de pulpe et/ou d'amande et/ou la pâte de pulpe de drupes.

L'invention couvre encore l'utilisation de l'huile de pulpe des drupes ou d'huile d'amande des drupes telle que décrite dans la présente description, incluant donc l'obtention par le procédé ou par l'appareil selon l'invention, comme produit alimentaire, cosmétique ou pharmaceutique.

La présente invention couvre encore l'utilisation de la pâte de pulpe de drupes fraîche, partiellement séchée ou déshydratée essentiellement déshuilée ou comportant toute son huile, comme aliment notamment pour l'alimentation humaine ou animale après désamérisation.

L'invention couvre encore l'utilisation des bois des noyaux, comme combustible, ou pour la fabrication d'un abrasif ou d'une charge, avantageusement après micronisation mécanique, ces bois étant de préférence tels qu'obtenus par le procédé ou par l'appareil selon l'invention tels que résultant de la description prise dans son ensemble.

L'invention couvre encore l'utilisation du tourteau d'amande comme source alimentaire et de principe actif tel qu'il est obtenu par le procédé ou par l'appareil selon l'invention tel que résultant de la description prise dans son ensemble.

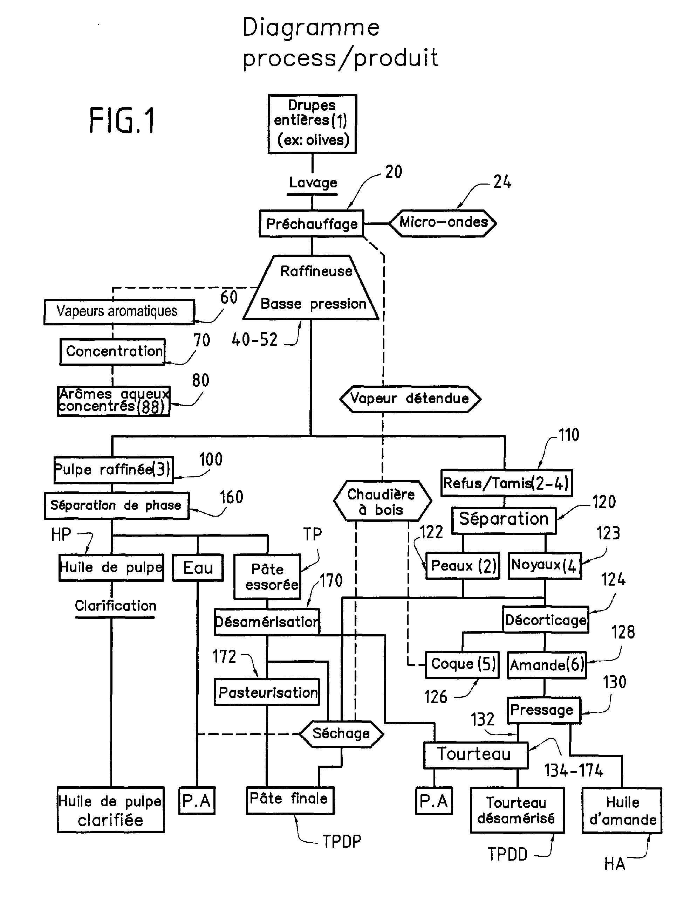

Il est à noter que les figures 1, 2 et 3 font partie intégrante de la présente invention et complètent ainsi la description. En outre, l'invention comprend aussi toute caractéristique qui apparaît être nouvelle par rapport à un état de la technique quelconque à partir de la description prise dans son ensemble, incluant les figures 1, 2 et 3 ces caractéristiques nouvelles sont revendiquées dans leur fonction et dans leur généralité.

L'invention sera mieux comprise dans ses avantages et ses caractéristiques à la lecture de la description qui va suivre faite en référence à un mode de réalisation actuellement préféré de l'invention faisant partie intégrante de l'invention dans sa généralité en référence aux figures 1, 2 et 3 annexées.

Dans les figures :
- la figure 1 représente un diagramme de principe des étapes de mise en oeuvre du procédé de traitement de drupes entières selon la présente invention pouvant être mis en oeuvre avec l'appareil objet de la figure 2 ou 3;
- la figure 2 représente schématiquement les parties essentielles d'un premier mode de réalisation d'un appareil de traitement des drupes entières selon la présente invention, référence générale 10, en coupe axiale verticale, avec un dispositif de séparation monté selon un axe horizontal.

La figure 3 représente schématiquement de manière similaire à la figure 2, un deuxième mode de réalisation d'un appareil selon l'invention, selon une variante du dispositif de séparation ici monté selon un axe de rotation sensiblement vertical, en coupe axiale verticale, les parties de l'appareil en aval de l'enceinte de séparation étant identiques à celles de la figure 2 et n'étant donc pas représentées.

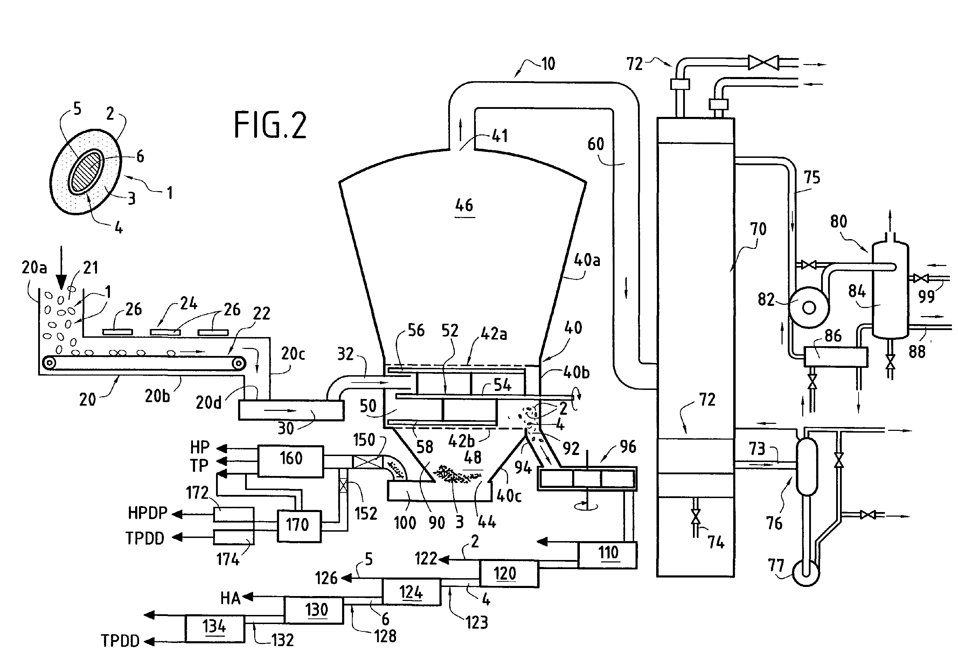

En référence aux figures 1 et 2, l'appareil de traitement de drupes entières selon la présente invention se caractérise par :
- les drupes entières 1 se composent de l'épicarpe ou peau 2, du mésocarpe ou pulpe 3 et de l'endocarpe ou noyau 4 se composant respectivement d'une enveloppe 5 vulgairement dénommée coque composée de fibres de bois et contenant une amande 6 à l'intérieur. Cette drupe entière est montrée de manière agrandie à la figure 1 afin de mieux comprendre sa structure.

Ces drupes entières sont généralement et de préférence fraîches, étant donné qu'elles contiennent une proportion supérieure en eau qui est aussi mise à profit dans le cadre de la présente invention comme il résulte de la définition du procédé et de l'appareil de la description précédente et de la description suivante en référence à l'appareil de la figure 2.

Les drupes entières 1 sont ainsi introduites, de préférence après lavage, dans une enceinte 20 de chauffage ou préchauffage qui peut comprendre une partie verticale 20a constituant en quelque sorte une trémie de réception et stockage des drupes entières 1, et une partie qui peut être, comme représenté, essentiellement horizontale 20b dans laquelle il est disposé un dispositif d'avancement des drupes entières, tel qu'un tapis roulant 22 transférant les drupes entières de la partie amont 20a vers une partie avale 20c ici verticale pouvant être ouverte en sa partie inférieure et comporter une ouverture d'évacuation 20d de transfert des drupes entières sortant de l'enceinte de préchauffage 20 en direction d'une enceinte tampon intermédiaire 30 qui sera décrite plus loin.

L'enceinte de préchauffage 20 est selon la présente invention pourvue de moyens de préchauffage 24 qui selon le mode de réalisation préféré représenté à la figure 2, comprennent plusieurs dispositifs émetteurs de micro-ondes 26, ayant pour fonction d'échauffer les molécules d'eau contenues dans le mésocarpe des drupes entières à une température prédéterminée qui sera avantageusement comprise entre 80 et 90°C. On peut, par exemple, utiliser une fréquence usuelle micro-ondes d'environ 2 450 MHz ou dans certains cas d'environ 915 MHz, sous réserve d'autorisation de cette fréquence.

En effet, cette température, comme cela ressortira de la description suivante, est préférée pour permettre ultérieurement la vaporisation rapide ou quasi-instantanée d'une fraction de l'eau de végétation.

Les drupes entières dont la pulpe a été préchauffée dans l'enceinte de préchauffage 20 sont recueillies dans l'enceinte intermédiaire 30 qui est prévue pour constituer une enceinte tampon ayant une fonction d'étanchéité par rapport à l'enceinte de préchauffage 20, l'étanchéité étant habituellement assurée par la présence d'un dispositif de transfert continue, de préférence à vis excentrée, formant tampon de matière, pour l'isoler de la pression atmosphérique régnant dans l'enceinte de préchauffage 20 grâce à l'ouverture d'entrée 21 des drupes entières.

Les drupes entières ayant leurs pulpes préchauffées sont ensuite transférées de l'enceinte intermédiaire 30 dans une enceinte 40 fermée sous pression réduite, par tout moyen de transfert bien connu de l'homme de l'art, par exemple par système de vis présent dans le conduit de transfert 32 aboutissant ici dans une partie 40b de l'enceinte sous pression réduite 40. L'enceinte 40 est subdivisée en trois parties suivantes :
a) La partie supérieure 40a de l'enceinte sous pression réduite 40 de préférence en forme de tronc de cône inverse définit une chambre 46 servant de chambre de détente des vapeurs émises sous pression réduite et entraînant les arômes volatils vaporisés qui sont transférés par une ouverture supérieure 41 communiquant avec un conduit de transfert 60 en direction d'un condenseur 70 également sous pression réduite dont le vide partiel est effectué par un groupe générateur de vide 80 bien connu de l'homme de l'art. La forme préférentiellement conique inversée de la partie supérieure 40a de l'enceinte a pour but de ralentir la vitesse des vapeurs émises, et donc d'empêcher les entraînements de particules solides.
   Dans le condenseur 70, le refroidissement est réalisé à l'aide d'un circuit de refroidissement 72 indirect à contre-courant classique, un dispositif de purge 74 étant prévu en bas du condenseur. L'eau condensée, sensiblement non aromatisée, recueillie en fond du condenseur 70 est soutirée par un dispositif 76 de soutirage et de récupération comportant aussi un conduit de réintroduction dans le condenseur 70 afin d'assurer une stabilisation du niveau hydraulique. Ce dispositif 76 comprend une pompe de circulation 77.
   Par ailleurs, en haut du condenseur 70, les vapeurs d'eau contenant les arômes concentrés sont soutirées par le conduit 75 en étant aspirées par le dispositif 82 tel qu'une pompe à vide à anneau liquide et introduits dans un dispositif de concentration 80, par exemple un séparateur de type cyclone 84 de manière à obtenir en fond du séparateur 84 des arômes aqueux concentrés soutirés par le conduit 88 qui peuvent être utilisés tels quels ou servir à aromatiser les huiles obtenues, telles que par exemple les huiles de pulpe ou les huiles d'amande, ou encore la pâte de pulpe obtenue par ailleurs. Il peut être prévu à un niveau intermédiaire un conduit 99 d'apport d'eau en début de cycle de fonctionnement. En outre, le sommet du séparateur formant cyclone 84 peut être en communication avec l'atmosphère. Il est aussi avantageusement prévu un dispositif 86 de circulation d'eau en circuit fermé refroidi alimentant la pompe 82 à anneau liquide.
b) la partie intermédiaire 40b délimitée par la présence de moyens de séparation respectivement supérieur 42a et inférieur 42b, de préférence constitués par des grilles de filtration formant tamis par exemple d'un cylindre perforé, ici fixe ou pouvant être rotatif, ayant des ouvertures comprenant soit des fentes soit des orifices de dimension appropriée pour laisser passer l'endocarpe ou pulpe des drupes et arrêter, pour réaliser leur séparation physique, les peaux et les noyaux des drupes.
   Dans la partie intermédiaire 40b de l'enceinte 40 est aussi disposé dans cet exemple un dispositif d'agitation favorisant une séparation physique entre les différents constituants de la drupacée entière telle que 52, par exemple comprenant ici un axe horizontal rotatif muni de pales racleuses 56, 58, coopérant avec le cylindre 42 qui ici est de position fixe. Les pales 56, 58 sont avantageusement de longueur décalée pour favoriser des effets de cisaillement favorisant la séparation physique de la pulpe relativement aux noyaux. Selon une variante, le dispositif d'agitation peut aussi comprendre un cylindre perforé 42 rotatif monté à rotation en étant solidaire de l'axe 54, tandis que les pales racleuses 56, 58 seraient fixes en étant alors solidaires des parois fixes de l'enceinte 40, de manière à obtenir les mêmes effets de raclage et de cisaillement.
c) La partie inférieure 40c de l'enceinte 40 définit une chambre 48 destinée à recueillir la pulpe passant à travers le cylindre 42 qui se transforme en une purée sous l'effet de l'évaporation quasi-instantanée de l'eau contenue dans la pulpe, aboutissant à une déstructuration des drupes entières avec éclatement de la pulpe. La purée ainsi formée comprend un mélange d'huile, de débris cellulaires de pulpe, d'eau, des peaux et des noyaux entiers.
   La purée de pulpe 3 récupérée dans la chambre 90 qui est filtrée par le cylindre 42 est recueillie au fond de la partie inférieure 40c de l'enceinte 40 et rassemblée dans une enceinte spécifique 100 de transfert, cette purée étant sensiblement ou essentiellement exempte des noyaux entiers et des peaux cireuses.
   Le mélange des peaux 2 et des noyaux 4, qui ne peut être filtré à cause de sa taille, est retenu et aussi séparé en aboutissant à une ouverture latérale d'évacuation 92 communiquant par un conduit 94 à un dispositif 96 de transfert, par exemple à pas rotatif.
   Le dispositif de transfert 96 constitue un système de pas rotatif assurant aussi une étanchéité similaire à celui de l'enceinte intermédiaire 30 de manière à préserver le vide partiel présent dans l'enceinte 40.

Dans le cadre de l'invention, il est avantageux que la partie supérieure 40a de l'enceinte 40 sous vide partiel présente une forme en tronc de cône inversé afin de limiter la vitesse d'entraînement des vapeurs. Le vide partiel régnant dans l'enceinte 40 est avantageusement inférieur à 100 hectopascals et de préférence compris entre 50 et 100 hectopascals.

Le mélange des peaux 2 et des noyaux 4 est ensuite envoyé depuis le dispositif 96 dans une enceinte intermédiaire de stockage 110 où ils peuvent être éventuellement séchés et stockés pour être traités plus tard en vue de leur séparation qui peut s'effectuer par tout moyen connu tel qu'un ventilateur cyclonique 120. On obtient ainsi, d'une part, les peaux 2 à la sortie 122 qui peuvent elles-mêmes être broyées pour être utilisées comme alimentation du bétail ou être traités spécifiquement pour en extraire les produits particuliers comme les cires.

En ce qui concerne les noyaux 4, ceux-ci sont envoyés par le conduit 123 à un dispositif de concassage ou broyage 124 avec des dispositifs bien connus de l'homme de l'art et qui permettent de séparer d'une part le bois 5 récupéré en 126 et d'autre part les amandes 6 à la sortie 128 qui restent ici entières avec leur pellicule protectrice.

Les bois des coques 5 sont récupérés en 126 et constituent une source énergétique pour l'entreprise qui permet de favoriser un bilan thermique permettant d'assurer l'auto-suffisance énergétique du procédé et de l'appareil.

Les amandes 6 récupérées par la sortie 128 peuvent être avantageusement envoyées à un dispositif de pressage 130 qui permet d'obtenir d'une part une huile d'amande HA qui peut être utilisée telle qu'elle dans l'alimentation, en cosmétique et en pharmacie, l'huile amande étant bien reconnue et par exemple dans le cas de l'huile d'amande d'olive étant obtenue proche de l'huile d'amande douce codex avec une présence de scalène qui lui est propre.

Après pressage dans le dispositif de pressage 130, les tissus restant après le pressage sont dénommés tourteaux et sont évacués en 132 pour être stockés en 134. Ces tourteaux 134 représentent environ 60 % des matières totales initiales et la richesse en protide (30 % mad) et sa composition en acide aminé le rende comparable aux tourteaux d'arachide et encore une source utilisable pour l'alimentation humaine et animale.

De plus, il est recommandé d'extraire dans le dispositif 134 un principe actif (P.A.) amer concentré qui est dans le cas du traitement d'olive comme drupes comprend principalement l'oléuropoéoside, qui est aussi présent dans les feuilles de l'olivier et bien connu en phytothérapie pour ses effets vasodilatateurs et hypotenseurs.

Par ailleurs, la pulpe 3 stockée dans l'enceinte de transfert 100, qui est essentiellement exempte des peaux et des noyaux, est transférée par tout dispositif de transfert et par exemple par une simple pompe, non représentée, dans un dispositif échangeur de type tubulaire tel que 150 notamment de façon à l'amener à la température adéquate pour des opérations d'extraction.

La pulpe 3 issue de l'enceinte 100 peut ainsi faire l'objet de séparation des phases aqueuse, huileuse et solide selon tout procédé de pressage, centrifugation ou décantation naturelle ou forcée dans le dispositif de séparation 160.

On doit noter que la purée de pulpe 3 est obtenue dans le cadre de l'invention, sans émulsion, ce qui augmente l'efficacité de son traitement.

A la sortie du dispositif de séparation 160 on obtient une huile pure de pulpe HP. On obtient aussi des tissus de pulpe partiellement déshuilés TP qui peuvent être éventuellement pasteurisés et/ou plus ou moins déshydratés.

Selon une variante de réalisation, la purée de pulpe 3 après passage dans le dispositif 150 peut être transférée par la vanne 152 dans un dispositif 170 de désamérisation plus ou moins importante selon la maturité du fruit pour subir ensuite une pasteurisation dans un dispositif de pasteurisation 172 pour former une huile de pulpe désamérisée pasteurisée HPDP, tandis que les tissus peuvent être soumis dans un dispositif de séchage 174 pour obtenir un tourteau de pulpe désamérisé déshydraté TPDD.

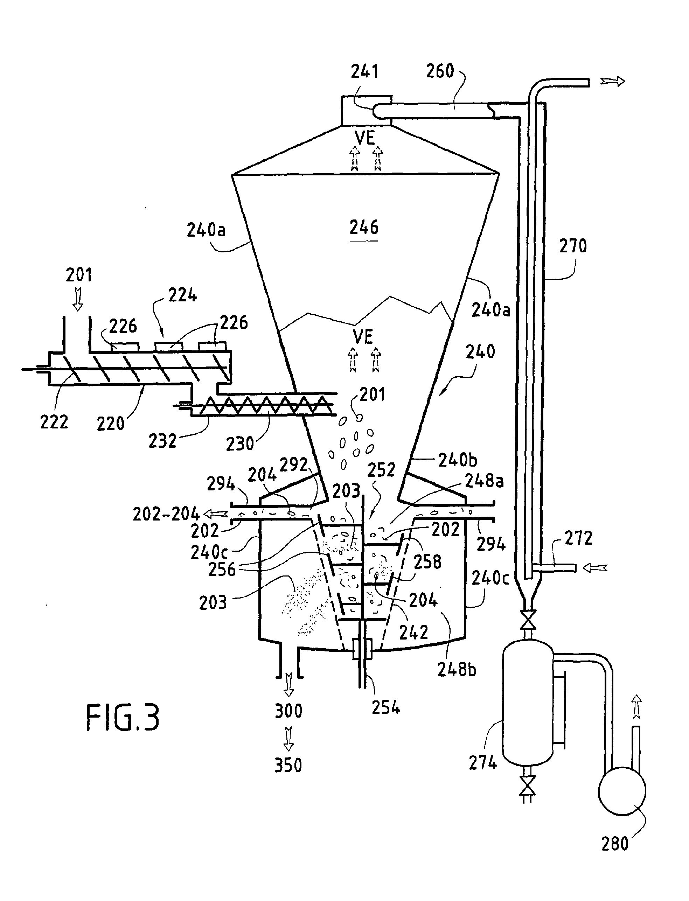

En référence à la figure 3, on a représenté un deuxième mode de réalisation d'un appareil de traitement des drupes entières selon la présente invention, qui a été modifié par rapport à l'appareil représenté à la figure 2 pour présenter un dispositif de séparation des noyaux et des peaux relativement à la pulpe, monté selon un axe sensiblement vertical, contrairement à celui de la figure 2 qui présentait un axe de rotation sensiblement horizontal. Par ailleurs , les parties en aval de ces dispositifs de séparation n'ont pas été représentées à la figure 3 et sont identiques à celles de la figure 2 afin de simplifier la figure. De ce fait, les mêmes chiffres de référence que ceux utilisés à la figure 2 ont été utilisé pour le mode de réalisation de la figure 3 pour les pièces identiques ou similaires, mais augmenté de 200 afin d'éviter toute interférence entre les chiffres de référence.

Ainsi:
- les drupes entières 201 sont ainsi introduites, de préférence après lavage, dans une enceinte 220 de chauffage ou préchauffage pourvu de moyens de chauffage ou préchauffage 224, par exemple dispositifs de chauffage ou préchauffage à micro-ondes 226 identiques à ceux du premier mode de réalisation de la figure 2. Dans l'enceinte de préchauffage 220, il est disposé un dispositif 222 d'avancement des drupes entières par exemple ici à vis sans fin, pour transférer les drupes entières en direction d'une enceinte tampon intermédiaire 230 similaire à l'enceinte tampon 30 de la figure 2, mais ici elle constitue en même temps le conduit d'introduction des drupes entières dans l'enceinte 240, similaire à l'enceinte 40, fermée sous pression réduite, et comprend donc le conduit 232 similaire au conduit 32.

Selon ce deuxième mode de réalisation, l'enceinte sous pression réduite 240 comprend une partie supérieure 240a similaire à la partie 40a de l'enceinte 40, une partie intermédiaire 240b située au voisinage du niveau d'introduction des drupes entières par le conduit 232, et une partie inférieure 240c similaire à la partie inférieure 40c de la figure 2

Dans ce mode de réalisation, on observera que les drupes entières sont introduites à un niveau intermédiaire de l'enceinte 240 sous pression réduite qui permet aux drupes entières de chuter sous l'effet de la gravité en étant ainsi physiquement fluidisées, en facilitant l'évaporation de l'eau présente dans les tissus de la pulpe pour produire une évaporation sous forme de vapeur d'eau symbolisée par les flèches en pointillé V E s'élevant à l'intérieur de l'enceinte et notamment dans sa partie supérieure 240a pour s'évacuer en direction du conduit d'évacuation 260 similaire au conduit 60 de la figure 2.

On constate ainsi que l'amélioration de ce mode de réalisation de la figure 3 par rapport à celui de la figure 2 réside dans la chute pendant quelques fractions de seconde des drupes entières à l'intérieur de l'enceinte au niveau de sa partie intermédiaire 240b en facilitant l'évaporation de l'eau contenue dans les pulpes qui a été préchauffée par le dispositif de préchauffage 220 et de manière précise par de préférence un dispositif à micro-ondes 224-226.

Comme il a été dit pour le premier mode de réalisation, l'évaporation quasi instantanée de l'eau contenue dans la pulpe produit une déstructuration des cellules de la pulpe.

Les drupes entières ayant leur eau quasi instantanément évaporée chutent dans la partie inférieure 240c de l'enceinte 240, définissant une chambre de séparation 248a, 248b, qui comprend un dispositif d'agitation 252 similaire dans sa fonction de celui du dispositif d'agitation 52 de la figure 2 mais qui est ici disposé avec un axe de rotation selon un axe sensiblement vertical au lieu d'être horizontal selon la figure 2. L'axe de rotation présente le numéro de référence 254 et correspond à l'axe de rotation 54 de la figure 2 et peut être solidaire des pales racleuses 258 et 256 similaires aux pales racleuses 58 et 56 de la figure 2, coopérant avec un filtre 242 solidaire de l'enceinte 240c, similaire au filtre 42 de la figure 2 qui est ici de préférence de forme tronconique inversée de manière à présenter une section plus étroite à sa partie inférieure, une section plus grande à sa partie supérieure, comme représenté. Les pales 256, 258, qui sont aussi avantageusement de longueur décalée comme pour le mode de réalisation de la figure 2, favorisent des effets de cisaillement induisant la séparation physique de la pulpe relativement au noyau. Comme pour le mode de réalisation de la figure 2, selon une variante, on peut inverser la partie fixe et la partie rotative, à savoir que la grille 242 de filtration peut être rotative et les pales peuvent être fixes.

Il est à noter que la grille 242 de filtration qui a la même fonction que la grille 42 de la figure 2 permet de filtrer d'une part la purée de pulpe 203 qui comprend la pulpe déstructurée, de l'eau, de l'huile de drupe; et, d'autre part, de retenir car non filtrés, les peaux 202 et les noyaux 204 qui, sous l'effet de l'agitation par les pales 258 sont progressivement rejetés vers le haut pour être évacués par l'ouverture 292 au travers du conduit 294 de manière similaire à l'ouverture 92 et au conduit 94 de la figure 2 pour être ensuite traités de manière identique à la figure 2. Cette évacuation des peaux 202 et noyaux 204 est facilitée par une pompe aspirante ou similaire.

Cette purée de pulpe 203, maintenant stockée dans la chambre 248b définie entre le filtre 242 et la paroi de l'enceinte 240c, qui comprend le mélange des cellules de pulpe déstructurées, d'huile de drupe, et d'eau, est soutirée à la base de la partie inférieure 240c de l'enceinte 240 par un conduit de soutirage aboutissant éventuellement à un dispositif de stockage intermédiaire 300 analogue au dispositif de stockage 100 de la figure 2 puis à un dispositif 350 analogue au dispositif 150 de la figure 2, le reste du traitement de séparation d'une part de l'huile de pulpe, de l'eau de la pulpe, et des tissus de la pulpe déstructurée étant les mêmes que ceux représentés à la figure 2.

Dans le cadre de l'invention, on peut observer dans la mesure où les équipements avals de l'enceinte sous pression réduite 40 ne seraient pas en mesure de permettre le traitement des quantités de drupacées entières récoltées, qu'il est possible d'extraire une partie aliquote de la purée 100 préparée entière de la suite du traitement pour la conserver au froid à une température comprise entre 2 et 4°C dans des bacs et sous atmosphère contrôlée afin de la traiter ultérieurement en permettant ainsi à l'industrie une plus grande indépendance vis-à-vis de la saisonnalité et de travailler sur une plus grande partie de l'année.

La conservation de cette purée est à la fois plus facile à stocker par suite de son encombrement réduit et permet une maîtrise de ses conditions de bonne conversation infiniment plus aisée que dans le cas des drupes entières.

On constate ainsi que l'invention permet de récupérer tous les constituants des drupes oléifères. L'appareil ainsi décrit et son procédé de mise en oeuvre dans les étapes essentielles rapportées à la figure 1 sont applicables à l'ensemble des drupes oléifères.

Dans le cadre de l'invention, les drupes oléifères préférées sont les olives, les drupes ou fruits du palmier à huile et les fruits ou drupes d'avocats.

Dans le cadre de la drupe ou fruit du palmier, on obtient tout d'abord une huile issue de la pulpe dénommée "huile de palme" d'extrême pureté et une huile issue de l'amande du noyau dénommée "huile de palmiste" tandis qu'avec l'avocat comme drupe, on obtient deux huiles d'avocat issues respectivement de la pulpe et de l'amande du noyau.

Il est à remarquer que pour l'avocat, le procédé et l'appareil selon l'invention permettent l'obtention d'une purée de pulpe entière dans des conditions rapides, continues et évitant l'oxydation et qui servent de base alimentaire pour un certain nombre de produits alimentaires humains spécifiques.

Enfin, la séparation préalable de la peau et du noyau relativement à la pulpe permet une valorisation de l'ensemble des co-produits tout en limitant la constitution d'effluents polluants liquides ou solides qui à terme deviennent un coût pour l'entreprise et/ou l'environnement.

Ainsi, l'invention permet de résoudre les problèmes techniques précédemment énoncés et d'aboutir à une solution constituant un progrès technique déterminant et particulièrement inattendu pour un homme de l'art.

Dans le cadre du mode de réalisation actuellement préféré du traitement d'olive comme drupe oléifère, les tableaux n°1 à 5 suivants donnent les masses des divers constituants, leurs proportions en poids, et le bilan thermique pour 100 kg d'olives entières fraîches traitées.

**TABLEAU n° 1**

| Composition moyenne des OLIVES (pour 100 kg) | | | | | | | | | | |
|---|---|---|---|---|---|---|---|---|---|---|
| | | Pulpe | | Peaux | | Amandes | | Coques | | Totaux |
| | kg | 41 | | 1,6 | | 0,3 | | 1,5 | | 40 à 50 |
| Eau | | | | | | | | | | |
| | % | | 55 | | 20 | | 15 | | 10 | |
| | kg | 18 | | 0,7 | | 0,7 | | 0,3 | | 18 à 25 |
| Huile | | | | | | | | | | |
| | % | | 24 | | 10 | | 35 | | 2 | |
| | kg | 16 | | 5,6 | | 1 | | 13,2 | | 30 à 40 |
| Solides | | | | | | | | | | |
| | % | | 21 | | 70 | | 50 | | 88 | |
| | kg | 75 | - | 8 | - | 2 | - | 15 | - | |
| Totaux | | | | | | | | | | 100 |
| | % | - | 100 | - | 100 | - | 100 | - | 100 | |

**TABLEAU n° 2**

| Pulpe après procédé traitement intégral (de 100 kg d'olives) | | | | | | | | | | | |
|---|---|---|---|---|---|---|---|---|---|---|---|
| | | Pulpe | | Peaux | | Amandes | | Coques | | Totaux | |
| | kg | 36 | | 1,5 | | 0,3 | | 1,5 | | 39 | |
| Eau | | | | | | | | | | | |
| | % | | 51 | | 19 | | 15 | | 10 | | 41 |
| | kg | 18 | | 0,8 | | 0,7 | | 0,3 | | 20 | |
| Huile | | | | | | | | | | | |
| | % | | 26 | | 10 | | 35 | | 2 | | 21 |
| | kg | 16 | | 5,6 | | 1 | | 13,2 | | 36 | |
| Solides | | | | | | | | | | | |
| | % | | 23 | | 70 | | 50 | | 88 | | 38 |
| | kg | 70 | - | 8 | - | 2 | - | 15 | - | 95 | - |
| Totaux | | | | | | | | | | | |
| | % | - | 100 | - | 100 | - | 100 | - | 100 | - | 100 |

**TABLEAU n° 3**

| Comparaison pulpe entière et après extraction | | | | | |
|---|---|---|---|---|---|
| Pour 100 kg d'olives | | Pulpe entière | | Pulpe déshuilée | |
| | kg | 32 | | 13 | |
| Eau | | | | | |
| | % | | 49 | | 43 |
| | kg | 18à20 | | 2à5 | |
| Huile | | | | | |
| | % | | 28 | | 7 |
| | kg | 15 | | 15 | |
| Solides | | | | | |
| | % | | 23 | | 50 |
| | kg | 68 | | 30 | |
| Totaux | | | | | |
| | % | | 100 | | 100 |

**Tableau n° 4**

| Produits issus du procédé de traitement intégral de l'olive | | |
|---|---|---|
| Produits | Poids | % eau |
| Huile de pulpe | 18 à 20 kg | 0% |
| Huile d'amande | 0,8 à 2 kg | 0% |
| Pulpe déshuilée | 30 à 35 kg | 30 % |
| Tourteau d'amande | 1,2 à 3 kg | 30% |
| Bois des noyaux | 17 à 23 kg | 20% |
| Arômes aqueux | 8 à 10 kg | 99 % |
| Peaux | 1,5 à 2 kg | 30% |
| Eaux de végétation | 20 à 25 kg | 100 % |

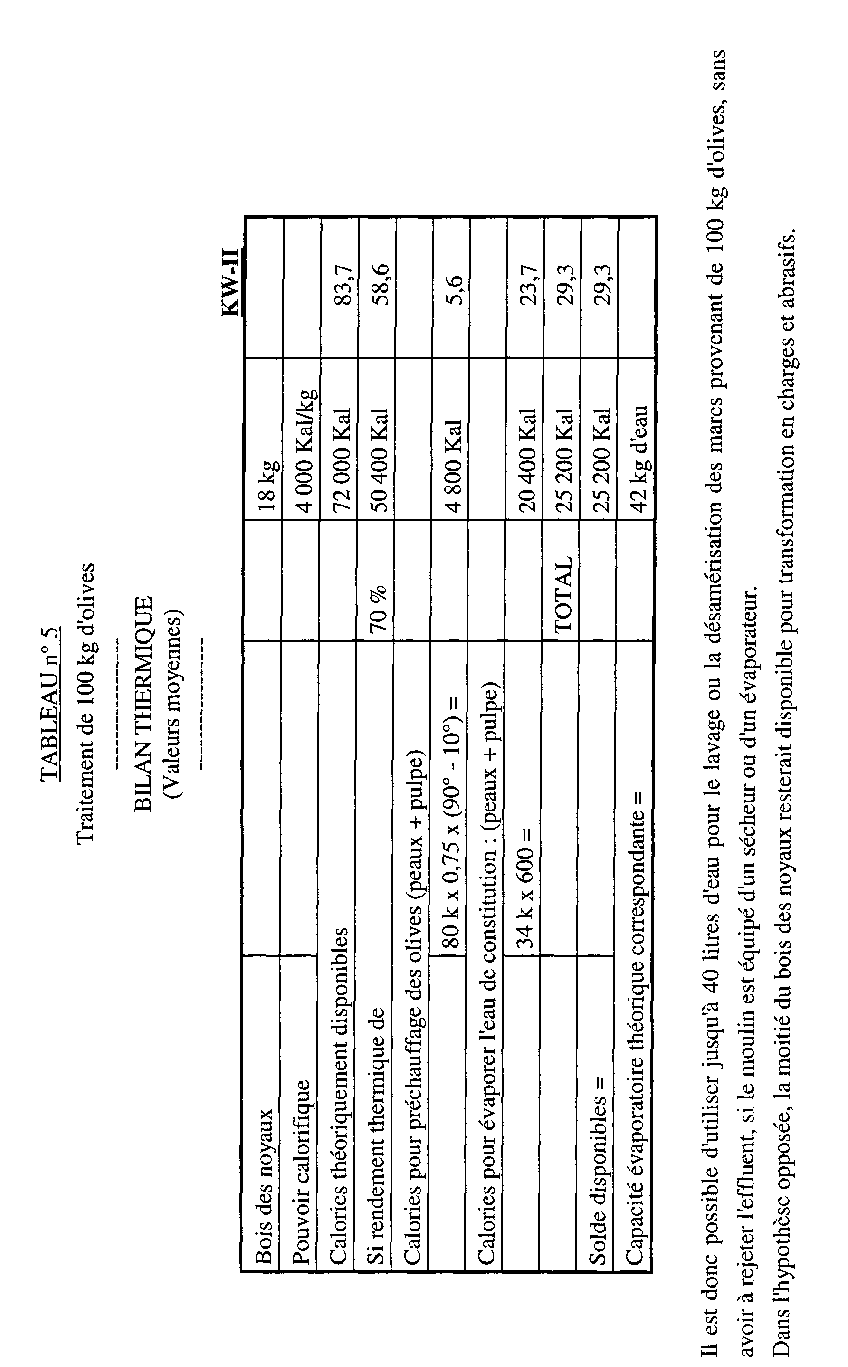

## Revendications

1. Procédé de traitement de fruis de type drupes oléifères comprenant un épicarpe ou peau, un mésocarpe ou pulpe, et un endocarpe ou noyau, lesdits fruits étant en particulier des olives, des fruits de palmiers oléifères ou des avocats, **caractérisé en ce qu'**il comprend :
a) un traitement de chauffage ou préchauffage des drupes entières dans des conditions contrôlées pour réaliser un chauffage ou préchauffage contrôlé limité à l'eau contenue dans la pulpe des drupes de préférence dans des conditions évitant sensiblement l'oxydation notamment principalement des antioxydants naturels présents dans la pulpe des drupes ;
b) un traitement réalisant l'évaporation rapide ou quasi-instantanée d'une fraction de l'eau préchauffée contenue dans la pulpe des drupes favorisant la déstructuration cellulaire limitée à la pulpe avec amorce de décollage de la pulpe relativement à la peau et au noyau, dans des conditions évitant sensiblement l'oxydation notamment principalement des anti-oxydants naturels présents dans la pulpe ;
c) la séparation physique et la récupération d'une part de la pulpe résultant de la déstructuration cellulaire due à l'évaporation partielle de l'eau précitée essentiellement exempte des noyaux et des peaux ; et d'autre part des noyaux essentiellement exempts de la pulpe, et avantageusement avec les peaux, de préférence dans des conditions évitant sensiblement l'oxydation ;
d) soit la récupération de la pulpe déstructurée contenant l'huile, essentiellement exempte des noyaux et des peaux pour une utilisation telle quelle, soit de préférence une séparation, de préférence dans des conditions évitant sensiblement l'oxydation, d'une part de l'huile de la pulpe et, d'autre part, de la pulpe déstructurée sous forme de purée partiellement déshuilée ;
e) de préférence la récupération de l'huile de la pulpe ainsi séparée, qui est essentiellement exempte d'huile issue des noyaux et des peaux, et contenant les anti-oxydants naturels de la pulpe, en améliorant ainsi la résistance à l'oxydation de ladite huile.

2. Procédé selon la revendication 1, **caractérisé en ce que** le traitement de chauffage ou préchauffage précité est réalisé dans une enceinte de chauffage, à l'abri de l'oxydation, dans un domaine de température et pendant une période de temps prédéterminés n'induisant pas sensiblement de risque de dégradation des pulpes, cette étape de chauffage ou préchauffage étant réalisée par tout système de chauffage direct ou indirect et préférentiellement par émission de micro-ondes.

3. Procédé selon l'une des revendications 1 à 2, **caractérisé en ce qu'**on réalise le préchauffage ou chauffage des drupes contrôlées limitées à l'eau contenue dans la pulpe des drupes par émission de micro-ondes, pour atteindre une température de l'eau contenue dans la pulpe suffisante pour favoriser une évaporation rapide ou quasi-instantanée d'une fraction de ladite eau lors de l'étape d'évaporation b précitée, de préférence le chauffage ou préchauffage est réalisé dans l'enceinte de chauffage ou préchauffage pour aboutir à une température de l'eau contenue dans le mésocarpe de l'ordre de 80°C à 90°C.

4. Procédé selon l'une des revendications 1 à 3, **caractérisé en ce que** les drupes entières chauffées ou préchauffées sont transférées de préférence immédiatement dans une enceinte d'évaporation maintenue à une pression inférieure à la pression atmosphérique, de préférence à une pression inférieure à environ 100 hectopascals, encore mieux comprise entre 50 et 100 HPa, où l'évaporation précitée d'au moins une partie de l'eau contenue dans la pulpe produit la déstructuration cellulaire précitée avec début de formation d'une purée contenant l'huile, avec refroidissement, et l'eau restante et des tissus cellulaires résultants de la pulpe éclatée ou désagrégée, ainsi que les noyaux entiers et les peaux.

5. Procédé selon l'une des revendications 1 à 4, **caractérisé en ce qu'**on sépare la pulpe déstructurée ou purée d'une part et des noyaux entiers et des peaux d'autre part dans un séparateur ou raffineuse avantageusement comprenant un tamis rotatif, de préférence à l'abri de l'air, notamment sous vide partiel ou atmosphère inerte, ledit séparateur ou raffineuse complétant avantageusement par agitation ou friction mécanique de l'affinage physique des tissus cellulaires de la pulpe et la libération et la coalescence des formations huileuses contenues dans la pulpe déstructuré, ainsi qu'avantageusement complétant la séparation physique des tissus déstructurés de la pulpe restant sur les noyaux.

6. Procédé selon l'une des revendications 1 à 5, **caractérisé en ce que** la pulpe entière, sous forme de purée résultant de sa déstructuration cellulaire, séparée des noyaux et des peaux, est soumise à une séparation de la phase solide, de la phase aqueuse si elle est encore présente et de la phase huile par tout système connu tel que pression, décantation, centrifugation pour obtenir ainsi une huile de la pulpe de drupes, essentiellement pure et essentiellement exempte d'huile de noyau, d'huile d'amande et d'huile de peau ainsi que sensiblement exempte des goûts et des arômes qui leur sont propres, avantageusement, cette séparation complémentaire est réalisée après passage de la purée de la pulpe entière dans un échangeur thermique contrôlant la température de la purée et/ou réalisant une déshydratation contrôlée complémentaire pour éliminer sensiblement complètement par évaporation la phase aqueuse restante.

7. Procédé selon l'une des revendications 1 à 6, **caractérisé en ce qu'**on réalise la séparation des noyaux d'une part et des peaux d'autre part des drupes par tout moyen physique de séparation, en particulier par tout dispositif approprié tel que procédé comprenant des mailles de dimension appropriée et/ou par des procédés à vibration et/ou ventilation notamment avec de l'air ; de préférence cette séparation ayant lieu après séchage des noyaux et des peaux.

8. Procédé selon la revendication 7, **caractérisé en ce que** les noyaux séparés des peaux sont ensuite broyés mécaniquement de façon à séparer les bois constituant la coque des noyaux de l'amande contenue dans les noyaux qui reste entière avec son épiderme protecteur ; avantageusement, on traite l'amande par tout système d'extraction utilisé pour extraire les huiles des graines ou amandes, en particulier par pression, pour obtenir une huile d'amande de drupe à haute valeur cosmétique et pharmaceutique d'une part et un tourteau formé par les tissus au moins partiellement déshuilés de l'amande à haute valeur alimentaire qui sont récupérés, ledit tourteau pouvant être encore traité pour un extrait et un principe actif à usage médicinal amère, qui dans le cas du traitement d'olives est dénommé oleuropéine.

9. Procédé selon l'une quelconque des revendications 2 à 8, **caractérisé en ce qu'**on récupère les composés les plus volatils de l'odeur naturelle des drupes oléifères qui se volatilisent dans l'enceinte d'évaporation maintenue sous basse pression, notamment en réalisant une condensation et une concentration en continue à la sortie de l'enceinte d'évaporation ; avantageusement, au moins une partie de ces composés les plus volatils ainsi récupérée, condensée et concentrée peut être éventuellement réintroduite dans le produit fini au goût des différentes clientèles.

10. Procédé selon l'une quelconque des revendications 1 à 9, **caractérisé en ce qu'**on traite des drupes oléifères choisies parmi le groupe consistant d'olives, des drupes de palmiers à huile et des avocats.

11. Appareil de traitement de drupes oléifères entières comprenant des peaux, de la pulpe et des noyaux contenant des amandes, par exemple des olives, des drupes de palmiers à huile et des avocats, **caractérisé en ce qu'**il comprend :
a) au moins un dispositif de préchauffage ou chauffage (20) des drupes entières (1) réalisant un chauffage ou préchauffage limité à l'eau contenue dans la pulpe, de préférence comprenant des moyens de chauffage à l'aide d'émetteurs micro-ondes, de préférence dans des conditions évitant sensiblement l'oxydation, notamment principalement des anti-oxydants naturels de la pulpe ;
b) au moins une enceinte fermée d'évaporation rapide ou quasi-instantanée d'au moins une partie de l'eau contenue dans les pulpes des drupes entières, dans des conditions évitant sensiblement l'oxydation, notamment principalement des anti-oxydants naturels contenus dans la pulpe, ladite évaporation rapide ou quasi-instantanée réalisant une déstructuration physique des tissus cellulaires de la pulpe avec décollage ou amorce de décollage de ces tissus cellulaires déstructurés relativement aux peaux et noyaux ;
c) au moins un dispositif (42-52) de séparation physique et de récupération d'une part (en 58-100) des pulpes essentiellement exempte des noyaux et avantageusement des peaux ; d'autre part (en 92-94-96) des noyaux essentiellement exempts de la pulpe, avantageusement avec les peaux, de préférence dans des conditions évitant sensiblement l'oxydation ; ce dispositif de séparation physique étant de préférence disposé dans ladite enceinte fermée ;
d) au moins un dispositif de séparation (160, 170, 172, 174) et de récupération de l'huile de la pulpe récupérée, essentiellement exempte de l'huile du noyau, et contenant les anti-oxydants naturels de la pulpe des drupes, ainsi qu'avantageusement d'une pâte de la pulpe sensiblement déshuilée ;
e) avantageusement au moins un dispositif de séparation des peaux d'une part et des noyaux d'autre part, après éventuel séchage avec avantageusement au moins un dispositif de broyage ou cassage mécanique des bois des noyaux dans des conditions maintenant l'intégrité de l'amande contenue dans les noyaux, cette amande restant entière dans son épiderme ; et au moins un dispositif de séparation mécanique des bois des noyaux d'une part des amandes, d'autre part.
f) avantageusement au moins un dispositif d'extraction de l'huile contenue dans lesdites amandes séparées, par exemple un dispositif de pression, avec récupération d'une part de l'huile d'amande d'une part et récupération d'un tourteau d'autre part qui peut de préférence être soumis à un dispositif d'extraction d'un principe actif amer qui, dans le cas du traitement d'olives, est dénommé oleuropéine.

12. Appareil selon la revendication 11, **caractérisé en ce que** le dispositif de préchauffage ou chauffage (20) des drupes entières (1) comprend des moyens de chauffage à l'aide d'émetteurs micro-ondes qui sont prévus pour réaliser un échauffement de l'eau contenue au moins dans la pulpe des drupes à une température comprise entre 80 et 90°C ; de préférence l'enceinte fermée précitée ou chaque dispositif précité étant tel que défini à l'une quelconque des revendications 2 à 10.

13. Huile de pulpe de drupacées et en particulier de l'olive de pureté améliorée, **caractérisée en ce qu'**elle est essentiellement exempte d'huile de noyaux contenue dans l'amande, **en ce qu'**elle contient les antioxydants naturels présents dans l'huile d'olive, notamment en étant préparée à l'abri de l'oxydation, en particulier à l'abri de l'oxygène de l'air, cette huile de pulpe d'olive étant susceptible d'être obtenue par le procédé de traitement tel que défini à l'une quelconque des revendications 1 à 10 ou par l'appareil de traitement d'olive tel que défini à la revendication 11 ou 12.

14. Huile de pulpe de drupacées et en particulier des olives selon la revendication 13, **caractérisée en ce qu'**elle contient aussi au moins en partie les composés les plus volatils de l'odeur naturelle du fruit, ou arômes, notamment en ayant été réintroduits dans l'huile finale, cette huile de pulpe contenant de préférence, la majeure partie des polyphénols naturels, et du tocophérol présent dans le fruit frais entier ; avantageusement la pure huile de pulpe d'olive présente une acidité exprimée en acide oléique inférieure à 1 % après 2 ans de stockage à température ambiante, de préférence une acidité exprimée en acide oléique inférieure à 0,7 g/100 g d'huile d'olive, après 2 ans de stockage à température ambiante.

15. Huile d'amandes de drupacées et en particulier des olives, telle que susceptible d'être obtenue par la mise en oeuvre du procédé selon l'une quelconque des revendications 1 à 10 ou de l'appareil selon la revendication 11 ou 12, avantageusement aromatisée avec au moins une partie des arômes naturels de la pulpe récupérés.

16. Pâte de pulpe de drupacées et en particulier des olives telle que susceptible d'être obtenue par le procédé selon l'une quelconque des revendications 1 à 10, ou par le procédé selon la revendication 11 ou 12, avantageusement aromatisée avec au moins une partie des arômes naturels de la pulpe récupérés.

17. Composés aromatiques volatils ou arômes récupérés des drupes et en particulier des olives, des drupes du palmier à huile ou des drupes d'avocat tels que susceptibles d'être obtenus par le procédé selon l'une des revendications 1 à 10 ou par l'appareil selon la revendication 11 ou 12.

18. Utilisation de l'huile de pulpe des drupes et en particulier de l'olive telle que définie à la revendication 12 ou 13, ou de l'huile d'amande des drupacées et en particulier de l'olive selon la revendication 14, comme produit alimentaire, cosmétique ou pharmaceutique.

19. Utilisation de la pâte de pulpe des drupes et en particulier de l'olive telle que définie à la revendication 15, fraîche, partiellement séchée ou déshydratée , et sensiblement déshuilée ou comportant toute son huile, comme aliment notamment pour l'alimentation humaine ou animale après désamérisation.

20. Utilisation des bois des noyaux, comme combustible, ou pour la fabrication d'un abrasif ou d'une charge, avantageusement après micronisation mécanique, ces bois étant de préférence tels qu'obtenus par le procédé selon l'une des revendications 1 à 10 ou par l'appareil de traitement selon la revendication 11 ou 12.

21. Utilisation du tourteau d'amande comme source alimentaire et de principe actif tel qu'obtenu selon l'une des revendications de 1 à 10 ou par l'appareil de traitement selon la revendication 11 ou 12.
